(19) **Europäisches Patentamt**
**European Patent Office**
**Office européen des brevets**

(11) **EP 1 478 732 B1**

(12) **EUROPEAN PATENT SPECIFICATION**

(45) Date of publication and mention
of the grant of the patent:
**10.09.2008 Bulletin 2008/37**

(51) Int Cl.:
*C12N 1/16* (2006.01)      *A23L 1/30* (2006.01)
*A61K 38/00* (2006.01)

(21) Application number: **03709671.6**

(22) Date of filing: **14.03.2003**

(86) International application number:
**PCT/DK2003/000167**

(87) International publication number:
**WO 2003/078605 (25.09.2003 Gazette 2003/39)**

(54) **A SELENIUM YEAST PRODUCT, A METHOD OF PREPARING A SELENIUM YEAST PRODUCT AND THE USE OF THE PRODUCT FOR PREPARING FOOD, A DIETARY SUPPLEMENT OR A DRUG**

SELEN-HEFEPRODUKT, VERFAHREN ZUR HERSTELLUNG EINES SELEN-HEFEPRODUKTS SOWIE VERWENDUNG DES PRODUKTS ZUR HERSTELLUNG VON LEBENSMITTELN, EINES NAHRUNGSZUSATZES ODER EINES ARZNEISTOFFS

PRODUIT LEVURE DE SELENIUM, SON PROCEDE DE PRODUCTION ET SON UTILISATION POUR PREPARER UN PRODUIT ALIMENTAIRE, UN COMPLEMENT ALIMENTAIRE OU UN MEDICAMENT

(84) Designated Contracting States:
**AT BE BG CH CY CZ DE DK EE ES FI FR GB GR HU IE IT LI LU MC NL PT RO SE SI SK TR**
Designated Extension States:
**LT LV**

(30) Priority: **15.03.2002 DK 200200408**

(43) Date of publication of application:
**24.11.2004 Bulletin 2004/48**

(73) Proprietor: **Pharma Nord ApS**
**6500 Vojens (DK)**

(72) Inventors:
 • **MOESGAARD, Sven**
 **DK-6051 Almind (DK)**
 • **PAULIN, Helge, Skousen**
 **DK-6580 Vamdrup (DK)**

(74) Representative: **Klinge, Ulla Callesen et al**
**Chas. Hude,**
**33, H.C. Andersens Boulevard**
**1780 Copenhagen V (DK)**

(56) References cited:
  **EP-A- 0 078 500**          **WO-A-98/37172**
  **US-A- 4 530 856**

 • **JULIAN E. SPALLHOLZ: "Nutritional, Chemical, and Toxicological Evaluation of a High-Selenium Yeast" INTERNAITONAL SYMPOSIUM ON SELENIUM IN BIOLOGY AND MEDICINE, PEKING, SELENIUM IN BIOLOGY AND MEDICINE: PROCEEDINGS OF THE THIRD INTERNATIONAL SYMPOSIUM ON SELENIUM IN BIOLOGY AND MEDICINE, HELD MAY 27-JUNE 1, 1984, XP002241158**
 • **"L-(+)Selenomethionine: A bioavailable Selenium Supplement" NEWSLETTERS OF SABINSA CORPORATION, JUNE-JULY 1999, [Online] XP002241159 Retrieved from the Internet: <URL:http://sabinsa.com/ news/n1r06_0799.ht mhttp: //www.lallemand.com/ANAH> [retrieved on 2003-05-14]**
 • **"Alkosel the bioavailability of selenomethionine" ANIMAL NUTRITION AND HEALTH, [Online] XP002241160 Retrieved from the Internet: <URL: http://www.lallemand.com/ANAH/eng/PDF s/ ALKO/ALKO.FS.ENG.042002.pdfhttp://www.la llemand.com/ANAH> [retrieved on 2003-05-14]**
 • **PONCE DE LEÓN C A ET AL: "Selenium incorporation into Saccharomyces cerevisiae cells: a study of different incorporation methods." JOURNAL OF APPLIED MICROBIOLOGY. ENGLAND 2002, vol. 92, no. 4, 2002, pages 602-610, XP002241161 ISSN: 1364-5072**

**Description**

Technical Field

The present invention relates to a selenium yeast product for use in food, dietary supplements or drugs, said product containing significant and homogeneous amounts of easily digestible and tolerable, organically bound selenium. The invention also relates to a method of preparing said selenium yeast product as well as the use of the selenium yeast product for preparing food, a dietary supplement or a drug.

Background Art

Selenium is an element essential to human nutrition. Selenium is ingested through the diet, which, however, has a varying content of selenium. In large parts of the world, crops with poor levels of selenium are cultivated because the presence of said element in the soil is modest.

The importance of selenium to humans has been substantiated through a great number of tests. Selenium is incorporated into different organic molecules, including in particular amino acids. L-selenomethionine, selenocysteine, selenocystine and selenoethionine are the most important compounds. Thus, selenium is part of proteins, which are of structural importance to the body. Furthermore, selenium is an important ingredient in a number of enzymes which influence the metabolism, the reproduction, the prevention of cancer, the immune defence and the psyche of humans. viz. Rayman, M., The importance of selenium to human health, Lancet 356:233-241 (2000).

Due to the often insufficient content of selenium in the ordinary diet, it is advantageous to add selenium in form of enrichment, dietary supplements or drugs. These may include an inorganic selenium such as selenite, or they may include organic sources, including selenium yeast. There is a significant difference between absorption and toxicity of inorganic and organic selenium, the inorganic compounds usually being absorbed significantly more slowly and also being more toxic than organic sources of selenium. An often used source of organic selenium is yeast with selenium content.

When cultivating yeast, it is possible to add the nutrient medium selenium in form of inorganic compounds, including sodium selenite and sodium selenate. The selenium added to the nutrient medium in this way is largely absorbed by yeast and incorporated into organic compounds, including L-selenomethionine.

Selenium yeast may be prepared by use of a number of yeast species, including *Saccharomyces cerevisiae, Saccharomyces boulardii sequela* and *Saccharomyces torula,* and by use of different cultivation conditions. As a result, a variable selection of organic compounds of selenium can be formed in the yeast. The reproducibility obtained by known methods can be unsatisfactory. A poor reproducibility has caused a reluctance to use selenium yeast by authorities, researchers and consumers. The Scientific Committee on Foods states that the L-selenomethionine content in selenium yeast varies between 20 and 50%, viz European Commission, Scientific Committee on Food, Opinion on substances for nutritional purposes which have been proposed for use in the manufacture of foods for particular nutritional purposes, viz. "PARNUTS", 12 May 1999, page 5. Scientific Committee on Food on the Tolerable Upper Intake Level of Selenium, 11 October 2000, page 2. It is of vital importance to the use in dietary supplements or drugs that the essential compounds of selenium, viz. species, are present in a homogenous concentration. This allows long-term studies of the importance of selenium to be carried out where the reproducibility from production to production is of essential importance for interpreting and using the results from the studies.

As a commercial product, selenium yeast is generally prepared by cultivating on molasses which have varying compositions. It is known from literature that different types of selenium yeast cause varying absorption levels and thus deviating responses, viz. Clausen, J. et al., A comparison of ten selenium supplementation products, Selenium in Medicine and Biology, Walter de Gruyter & Co 305-14 (1988). A number of patents describe the cultivation of selenium yeast containing different concentrations of selenium. Thus, US Patent 4,530,849 discloses the cultivation of selenium yeast with approx. 1000 ppm of selenium. However, the patent does not disclose how the selenium is bound in the yeast or to which extent the method can be reproduced.

Patent application WO 98/37172 relates also to the cultivation and the use of selenium yeast. The method by cultivation includes the steps of admixture of a watersoluble salt of selenium with a nutrient medium followed by the addition of an aqueous suspension of yeast. The resulting selenium yeast has a selenium content which is not divided into constant, homogeneous compounds. The possibility of obtaining a homogeneous yield and composition of selenium compounds is not described either.

It is a known fact that yeast and other microorganisms can be cultivated on a medium containing a minimum level of nutrients, a so-called minimal medium. However, such a method is not usually used industrially because it might result in a poor yield and an undesired composition of the microorganisms. Thus, it is surprising that it is possible by cultivation on a minimal medium in accordance with the present invention to obtain a good yield and a reproducible composition of selenium yeast which is optimal in human nutrition.

Thus, the present invention relates to a method of preparing a yeast product containing significant and homogeneous amounts of digestible, organically bound selenium. The resulting yeast is a powder which may be used directly or compressed into tablets by conventional techniques. These tablets can be marketed as food, dietary supplements or drugs.

Brief description of the Invention

In a first aspect, the invention thus relates to a selenium yeast product for use in food, dietary supplements or drugs, said product being characterised by having a content of organic selenium compounds corresponding to between 1000 and 1600 ppm, preferably between 1100 ppm and 1500 ppm, most preferably between 1200 ppm and 1400 ppm of selenium, and by the content of L-selenomethionine constantly constituting at least 55% of the total selenium content, and by the content of selenium in inorganic selenium compounds not exceeding 1% of the total selenium content.

The resulting product is furthermore characterised by a human absorption of more than 85%.

In a second aspect, the invention relates to a method of preparing a selenium yeast product for use in food, dietary supplements or drugs, whereby the yeast is cultivated under aerobic conditions, said method being characterised by

i) nutrients being fed to the yeast during the cultivation to an extent corresponding to the consumption of said nutrients in the yeast;

ii) glucose and/or maltose being the only sources of carbon in the feeding medium;

iii) the concentration of ethanol during the cultivation not exceeding 1%, preferably 0.5% and most preferably 0.2%;

iv) the pH value during the cultivation being maintained at between 4.0 and 6.0, preferably between 4.4 and 5.7, most preferably between 4.7 and 5.4, such as 5.0; and

v) the feeding medium being mixed with an aqueous salt of selenium in an amount corresponding to between 1000 and 1500 ppm of selenium, calculated on dry matter in the yeast.

In a third aspect, the invention relates to a selenium yeast product prepared by the method of the invention.

In a fourth aspect, the invention relates to food, a dietary supplement or a drug including a selenium yeast product according to the invention.

In a fifth aspect, the invention relates to the use of the selenium yeast product according to the invention for preparing food, a dietary supplement or a drug.

Detailed description of the invention

Prior to the detailed description of the different embodiments of the invention, a number of relevant definitions specific to the main aspects of the invention are provided below.

Definitions

Minimal medium: Medium containing the minimum amount of nutrients necessary for obtaining yeast growth.

Pharmaceutical quality: Properties of products described in a national pharmacopoeia.

Starting medium: Mixture of water and additional nutrients seeded with yeast.

Feeding medium: nutrients added to the starting medium/culture subsequent to the pitching of yeast.

The Zak-method: Method of cultivating yeast, whereby said yeast is pitched to a starting medium followed by nutrients being added under aerobic conditions through a feeding medium. The addition of nutrients is carried out under aerobic conditions at a rate corresponding to the absorption rate of said nutrients in the yeast. A correct control results in a formation of only a small amount of alcohol. The alcohol formed is consumed by the yeast in the last stage of the cultivation. The method is conventional and is referred to as the Z-method. It is as a principle developed by Mr Soren Sak and described in Danish patent No 28507 (1921).

Human absorption: Difference between ingested amount of isotope and excreted amount of isotope in defecation. Human absorption is calculated in percent of the ingested amount of isotope.

As mentioned above, hitherto known methods of preparing selenium yeast products use primarily molasses as a carbon source and when a glucose based medium is used, cf. WO 98/37172, this method deviates from the method of the invention by not ensuring a continuous addition of nutrients and selenium which corresponds to the cell growth.

In addition, an admixture of selenium is carried out within a relatively short period of time, which complicates the formation of organic compounds of selenium, which have particular importance in the human nutrition.

The nutrients for the cultivation of yeast according to the invention include sources of carbon and nitrogen as well as micro nutrients in form of vitamins and minerals. The carbon must be in a form which can be ingested immediately during the cultivation and therefore it must be highly soluble in water and have a composition allowing a consumption by means of the enzymes present in the yeast. These carbon sources include glucose and maltose which can be purified into glucose syrup in such a manner that they can be used as nutrients for microorganisms producing food, dietary supplements or drugs. As a source of nitrogen, it is possible to use inorganic compounds, including ammonia with a sufficiently high purity level so as to avoid toxicity or formation of undesired compounds. The metabolism of the yeast is not completely known and consequently it is necessary to add micro nutrients in form of yeast extract, which is described in pharmacopoeia.

Thus, yeast can according to the invention be prepared on the basis of raw materials of a pharmaceutical quality and composed in such a manner that the yeast has a minimum level of nutrients which can be used for growth. A typical method of cultivating the yeast includes the following steps:

1) Processing the nutrient medium
2) Seeding with pitching yeast
3) Cultivating
4) Harvesting
5) Washing
6) Heat treating
7) Drying

The nutrient medium is produced by dissolving sugar substances, vitamins and minerals in water heated to between 24 and 37°C, preferably between 26 and 34°C, and best between 28 and 32°C prior to the pitching of yeast.

A seeding with pitching yeast is carried out to the complete and heated nutrient medium by suspending the yeast cells in the medium while stirring at a frequency of between 1 and 2 Hz. The selenium yeast can be prepared by use of a number of species, including *Saccharomyces cerevisiae, Saccharomyces boulardii sequela* and *Saccharomyces torula.* Among said species, *Saccharomyces cerevisiae* is generally considered to be suitable for human ingestion, and it is widely used for the preparation of bread and alcohol. For the preparation of a selenium yeast product according to the invention it is an advantage to use a production strain, which is genetically stable and thus less inclined to be subjected to a mutation. In an embodiment according to the present invention, the strain ATCC No 74366 is thus used without, however, limiting the invention thereto.

The cultivation is carried out on the basis of a nutrient medium which is produced as a particular minimal medium and seeded with the above pitching yeast. Furthermore, additional carbohydrates are added in form of glucose and/or maltose. An aqueous solution of ammonia is admixed as a source of nitrogen. In order to ensure the highest possible growth and to counteract the formation of alcohol, large amounts of air are introduced, preferably atmospheric air containing sufficient oxygen. In large scale preparations, it is in practice difficult to ensure a complete oxidation, which can change the nutrition need of the yeast slightly. Cultivation is terminated at a concentration of yeast of approximately 4% by weight, calculated on the total content of nutrient medium and yeast. The addition of nutrients is carried out under aerobic conditions at a rate corresponding to the absorption rate of said nutrients in the yeast. As a result, only a small amount alcohol is formed through a correct control. The alcohol formed is consumed by the yeast in the last stage of the cultivation.

In practice, the addition of nutrients in the correct amount is controlled by continuously measuring the amount of alcohol in form of ethanol formed during the growth. If the nutrients are added too slowly, no alcohol is formed and if the addition is carried out too fast, significant amounts of alcohols are formed. Therefore, the amount of alcohol present at any time should not exceed 1%, preferably 0.5% and most preferably 0.2%. Furthermore, the pH of the growth medium can be used as an indicator for maintaining the correct balance between consumption and addition of nutrients, and therefore the pH is controlled and adjusted during the growth so as to maintain the pH between 4.0 and 6.0, preferably between 4.4 and 5.7, most preferably between 4.7 and 5.4, such as 5.0.

The harvest involves a separation of the cultivated yeast from the nutrient medium. This separation is best achieved by centrifuging, whereby a concentrated yeast cream is produced containing approximately 20% by weight of yeast.

The purpose of a washing is to remove the excess of nutrients in the yeast cream. The washing is most advantageously carried out by adding water followed by a centrifuging so that a yeast cream is produced. In this way, the yeast is washed 2 to 6 times and preferably 4 times, whereby practically all extracellular nutrients are removed from the yeast cream.

The purpose of the heat treatment is to kill the yeast cells so that the selenium present therein becomes available for the human digestion. A heat treatment results also in an extensive disintegration of the yeast cells. An advantageous heat treatment can be carried out as a pasteurization in a plate heat exchanger at a temperature of 87°C and a standing time of 30 seconds.

The yeast can be dried by conventional methods of drying organic material including freeze drying, drum drying, tray drying or spray drying, preferably spray drying. A spray drying removes water at an input temperature for air of between 160 and 240°C, preferably between 180 and 220°C and best about 200°C. The output temperature can in this connection be from 70 to 90°C, preferably 80 to 90°C and best about 86°C. The resulting powder has a water content of between 4 and 9%, preferably between 6 and 9% and best about 8%. The powder can subsequently be treated by way of moistening with water and followed by a drying so as thereby to improve the capability to absorb water later on.

Selenium yeast according to the invention can be used for preparing food, dietary supplements or drugs, either as the only source of selenium or in combination with other selenium containing ingredients.

Thus, the invention also relates to food, a dietary supplement or a drug, which as a source of selenium uses the selenium yeast according to the invention.

In an embodiment of the invention, the use includes a product including a disintegrating agent, a flow agent as well as selenium yeast. This mixture is compressed into a tablet containing between 25 and 800 jug of selenium, particularly between 40 and 300 $\mu$g of selenium and especially 50 to 200 $\mu$g of selenium per tablet.

However, in addition to the scope of application described above the invention can also be used for other types of food to be enriched with selenium such as flour, other powdery food as well as drinks.

The invention is explained in detail below with reference to the following examples.

EXAMPLES

EXAMPLE 1

1a)

In this example, a fermentor with a volume of 0.014 $m^3$ is used.

At the beginning of the cultivation, a nutrient medium is prepared with the following composition of raw materials of a pharmaceutical quality:

| | | |
|---|---|---|
| Water | Ph.Eur. | 5,400 g |
| Glucose syrup | Ph.Eur. | 31.8 g |
| $KH_2PO_4$ | Ph.Eur. | 25 g |
| Ammonia water 2.5% | Ph.Eur. | 114 g |
| Biotin (0.01%) | Ph.Eur. | 2.1 ml |
| Thiamine hydrochloride (1.0%) | Ph.Eur. | 2.5 ml |
| Calcium pantothenate | Ph.Eur. | 0.08 g |
| Yeast extract | USP | 75 g |
| Iron sulphate | Ph. Eur | 0.10 g |
| Magnesium sulphate | Ph.Eur. | 5.0 g |
| Manganese sulphate | Ph.Eur. | 0.033 g |
| Zinc sulphate | Ph. Eur | 0.033 g |

Subsequent to adjusting the temperature at 30°C, 5 g of pitching yeast is admixed. The seeded nutrient medium is blown through with sterile atmospheric air in an amount of 20 litres per minute. A stirring is carried out by means of a spindle at a frequency of 17 Hz. Sulphuric acid is used to adjust the pH so as to maintain the pH at between 4.7 and 5.4. It is controlled that ethanol does not exceed 0.2%, and in addition the concentration of ethanol is maintained as close to 0 as possible.

During the cultivation, nutrients are added with the feeding medium in the following amounts:

| | | |
|---|---|---|
| Glucose syrup | Ph. Eur | 1,060.5 g |
| Ammonia water 2.5% | Ph. Eur. | 1,451 g |

In addition, sodium selenite is admixed in ammonia water 2.5%, as follows:

Sodium selenite    Ph. Eur    1.2758 g

Glucose syrup, ammonia water and sodium selenite are added at a rate corresponding to the consumption rate of the substances in the yeast. This is controlled by continuously measuring the formation of alcohol. The concentration of alcohol is maintained close to 0.

The addition of ammonia water (2.5%) is terminated after 18 hours.
After 19 hours, the cultivation is terminated.

The harvest of the yeast is carried out by transferring the medium with the yeast to a centrifuge wherein the yeast cream is separated from the excess medium within 5 minutes. The resulting yeast cream with a dry matter content of approx. 20% is admixed 5,000 g of water. Subsequently, the yeast mixture is centrifuged again. The washing water is removed and the resulting yeast cream is admixed 5,000 g of water. The process is repeated 4 times to separate the yeast cells from the medium.

The washed yeast cream is carried through a plate pasteurizer in which it is subjected to a heat treatment at 87°C with a standing time of 30 seconds. Immediately after the heat treatment, the yeast cream is cooled to 4°C in a plate pasteurizer. The amount of yeast cream is 1,897.5 g.

The yeast cream is transferred to a freeze dryer. Subsequent to freezing at -40°C for 24 hours, the water sublimates within 18 hours. 380 g of dried selenium yeast is hereby provided having a water content of 0.2% and a concentration of selenium of 1,380 ppm in dry matter. The dietary properties and the reproducibility of the resulting selenium yeast product prepared are determined in the manner described below in Examples 2, 4 and 5.

1b)

Yeast is prepared in accordance with Example la above. However, a fermentor with a volume of 150 m$^3$ is used. The nutrient medium is composed of corresponding ingredients in the same mutual proportions and has a total weight of 56,848 kg.

Glucose syrup and ammonia water 2.5% are added in the same way as in Example 1 a. For this purpose, 13,500 kg of glucose syrup and 1,410.5 kg of ammonia water 25% are used. Selenium in form of sodium selenite is admixed in an amount of 15 kg. The addition of ammonia water is terminated after 18 hours and the cultivation is terminated after 19 hours.

The washing and the heat treatment of the yeast are carried out according to the principles described in Example 1a.

The drying of the yeast is carried out by spray drying in a drying tower having a rotating sprayer wheel. The frequency of the sprayer wheel is set at 167 Hz. The air temperature for drying is set at 200°C. The resulting starting temperature is 86°C. The total amount of powder with a water content of 7% is 5,035 kg. The concentration of selenium is 1,355 ppm on dry matter. The dietary properties and the reproducibility of the resulting selenium yeast product prepared are determined in the manner described below in Examples 2, 4 and 5.

EXAMPLE 2

Digestibility and absorption of selenium yeast.

2 a) In vitro digestibility of selenium

As a basis for determining the in vitro digestibility of selenium yeast, a further development of the method 9.1 of 15 November 1994 of The Danish Plant Directorate for determining the enzyme-digestible organic matter in pigs, viz. EFOS pigs, is used. The method has been changed so as to correspond to the conditions of the human digestion. Enzymes, which can decompose cellulose, are thus not included in this in vitro study.

The principle is treatment with pepsin followed by treatment with pancreatin. Undissolved sample material is filtered off and dried. By comparing the determinations of selenium in the original sample with the content in the filtrate and the retentate, the digestibility of selenium is calculated.

Thus, selenium yeast is mixed at pH 2.0 with pepsin and incubated at 40°C for 75 minutes followed by treatment with pancreatin at pH 6.8 at 40°C for 3 hours and 30 minutes. Subsequently, a filtration is carried out by means of vacuum and the selenium content in the filtrate and the retentate, respectively, is determined. The selenium content in the filtrate in percent of the total selenium content in the sample is an expression of the in vitro digestibility of selenium yeast.

A selenium yeast is obtained with the following characteristics:

Digestible selenium: 99%

2 b) In vivo absorption and retention of selenium

As a basis for determining the in vivo digestibility, selenium yeast according to the invention is administered where a stable isotope having a content of selenium-77 of 99.3% is used for the cultivation. Since naturally occurring selenium only contains 7.8% Se-77 and at the same time contains 49.82% of Se-80, it is possible to determine the proportion in human material originating from such an addition of isotope by measuring these isotopes via ICP-MS, viz. Inductively Coupled Plasma - Mass Spectrometry.

The determination of in vivo absorption, retention and bioaccessibility is moreover carried out as follows:

Twelve male participants aged 20 to 55 are administered a single dose of selenium yeast corresponding to 300 $\mu$g selenium-77. The participants collect faeces and urine for a period of 5 days, and 11 blood samples are collected during said period. In order to control the collection of faeces and urine, PABA, viz. para-amino-benzoic-acid, and plastic tubes, respectively, are administered which can be recovered in both the urine and the faeces as a control of the collection. The absorbed amount of selenium-77 is determined by comparing the ingestion of selenium-77 with the extraction through faeces.

The retention, viz. the retained amount, is determined as administered trace amount minus excretion through faeces and urine compared to the administered trace amount. The blood samples are used to describe the pharmaco-dynamics.

The following characteristics for absorption and retention of the selenium yeast according to the invention are obtained:

The absorption is 89%. The retention is 74%.

2 c) In vivo dosage/response at long-term ingestion

A blank experiment was carried out for a continuous period of 2 years. A group of 49 persons ingested a tablet each day with a content of 0, 100, 200 or 300 $\mu$g of selenium in form of selenium yeast according to the invention. Furthermore, the participants ingested through their food the amount of selenium naturally occurring in the diet, viz. approximately 50 $\mu$g/day. After 2 years where a balance between absorption and excretion has been obtained, the selenium content in whole blood was determined.

The following results were found:

| Ingestion per day ($\mu$g) | Number of participants | Mean value of selenium in whole blood ($\mu$g/l) | Absorption of selenium per $\mu$g ($\mu$g /l/ $\mu$g) | Increase compared to placebo (%) |
|---|---|---|---|---|
| 0 | 17 | 95.6 | - | 0 |
| 100 | 11 | 177.2 | 0.816 | 85 |
| 200 | 8 | 307.6 | 1.06 | 222 |
| 300 | 13 | 440.8 | 1.15 | 361 |

Based on the above results, a linear connection between the ingested dosage of selenium yeast and selenium content in whole blood could be determined and expressed in the following way:

$$\text{Selenium in whole blood } (\mu\text{g/l}) = 1.12 \text{ x ingestion } (\mu\text{g/day}) + 38$$

The discovered response on selenium yeast according to the invention surpasses the prior art description of selenium yeast, viz. Schrauzer, G. N., Selenium in human nutrition, Bioinorganic Chemistry, 8:303-318 (1978).

2 d) Measurement of side effects at long-term ingestion

For a period of 1-2½ years, selenium yeast according to the invention was administered in doses of 100, 200 or 300 $\mu$g or placebo to 806 persons in 4 equally divided groups. The period covers approximately 1400 person-years.

During this period, the tolerance and the side effects were tested in all persons, 2.6%, viz. 21 of the 806 persons, reported side effects. Before the randomization was terminated, the side effects were categorised as either mild, 17 persons, moderate, 4 persons, or serious, 0 persons.

Subsequent to divulgating the randomization, the side effects turned out to be divided randomly on the groups, 8 relating to placebo, 8 to 200 $\mu$g Se and 6 to 300 $\mu$g Se. The conclusion was that the side effects were insignificant and did not relate to the selenium yeast according to the invention.

EXAMPLE 3

Tablet:

Preparation of tablet of selenium yeast according to the invention is carried out by means of the following ingredients:
Selenium yeast according to the invention

| | |
|---|---|
| Tablet auxiliaries: | microcrystalline cellulose silicon dioxide magnesium salts of fatty acids |
| Flow agent: | di-calcium phosphate |
| Surface-treatment agent: | hydroxyl propyl methyl cellulose talcum |
| Colorant: | titanium dioxide |

The ingredients are mixed in a conventional manner and compressed into tablets so that each tablet contains 100 $\mu$g of selenium originating from selenium yeast.

EXAMPLE 4

Speciation of selenium yeast

A selenium yeast cultivated in a conventional manner by use of molasses and 2 batches of selenium yeast according to the invention was examined for content of various compounds of selenium. The samples were subjected to acid hydrolysis with thioglycolic acid as stabilizer in oxygen-free surroundings. The content of selenomethionine and sodium selenite was then determined by HPLC against laboratory references.

The selenium yeast cultivated on molasses had a content of 49% of selenomethionine of the extractable and HPLC-accessible part, while the selenium yeast according to the invention in two independent production batches showed a content of 72.8 and 72.9%, respectively, of selenomethionine of the extractable and HPLC-accessible part.

The extractable part of total selenium exists as 95 to 101%, of which approximately 80% can be tested by means of HPLC. The minimum true amount of 1-selenomethionine of total selenium is thus 72.8% x 95% x 80% = 55.3%. The highest possible true amount is approx. 90%. The measured amount of sodium selenite is less than 1%. It is possible to use a method described by Erik. H. Larsen et al., viz. Larsen, E. H. et al., J. Anal. At. Spectrom., 16, 1403-1408, 2000, for determining species of selenium.

In the Example, importance is attached to the reproducibility rather than to the actual true amount.

EXAMPLE 5

In vivo absorption of selenium yeast and inorganic selenium

In the same way as in the application Example 2b, either selenium yeast according to the invention or inorganic selenium-77 was administered to the same 12 test persons. The human absorption of the two sources of selenium was 89% for selenium yeast according to the invention and 23 % for inorganic selenium, respectively.

**Claims**

1. A method of preparing a selenium yeast product for use in food, dietary supplements, or drugs, whereby said yeast is cultivated on a minimal medium under aerobic conditions, **characterised by** the steps of:

   a) cultivating the yeast, which includes

i) nutrients and selenium being fed to the yeast during the cultivation to an extent corresponding to the consumption of said nutrients and selenium in the yeast;

ii) glucose and/or maltose being the sole sources of carbon in the feeding medium;

iii); the concentration of ethanol during the cultivation not exceeding 1%, preferably 0.5% and most preferably 0.2%;

iv) the pH value during the cultivation being maintained at between 4.0 and 6.0, preferably between 4.4 and 5.7, most preferably between 4.7 and 5.4, such as 5.0; and

v) an aqueous salt of selenium being admixed to the feeding medium in an amount corresponding to between 1000 and 1500 ppm, of selenium, calculated on dry matter in the yeast;

b) isolating the yeast obtained in step (a).

2. A method according to claim 1, **characterised by** the isolation including harvest by way of centrifuging or filtration.

3. A method according to claim 1, **characterised by** further including the steps of:

c) washing the yeast cells from step (b),
d) heat treating the yeast cells from step (c), and
e) optionally drying the product from step (d).

4. A method according to any of claims 1 to 3, **characterised by** the minimal medium being composed of raw materials of a pharmaceutical quality.

5. A method according to any of claims 1 to 4, **characterised by** the yeast including a species of the genus *Saccharomycetaceae,* preferably *Saccharomyces cerevisiae, Saccharomyces boulardii sequela* and/or *Saccharomyces torula.*

6. A method according to claim 5, **characterised by** the yeast being *Saccharomyces cerevisiae.*

7. A selenium yeast product **characterised by** being obtainable by the method according to any of claims 1 to 6.

8. A use of the selenium yeast product according to claim 7 for preparing a food product.

9. A use of the selenium yeast product according to claim 7 for preparing a dietary supplement.

10. A use of the selenium yeast product according to claim 7 for preparing a drug.

**Patentansprüche**

1. Verfahren zum Herstellen eines Selenhefeprodukts zur Verwendung in Nahrungsmitteln, Nahrungsmittelergänzungen oder Arzneimitteln, wobei die Hefe unter aeroben Bedingungen auf einem Minimalmedium gezüchtet wird, **gekennzeichnet durch** die Schritte des

a) Züchtens von Hefe welche umfasst

i) Nährstoffe und Selen, welche der Hefe während der Zucht in einem Maße zugeführt werden, das dem Verbrauch der Nährstoffe und Selen in der Hefe exakt entspricht;

ii) Glukose und/oder Maltose die einzigen Kohlenstoffquellen in dem Zuführmedium sind;

iii) die Ethanolkonzentration während des Züchtens 1 %, bevorzugt 0,5 % und am bevorzugtesten 0,2 % nicht übersteigt;

iv) der pH-Wert während des Züchtens zwischen 4,0 und 6,0, bevorzugt zwischen 4,4 und 5,7, am bevorzugtesten zwischen 4,7 und 5,4, wie beispielsweise bei 5,0, gehalten wird; und

v) ein wässriges Selensalz dem Zuführmedium in einer Menge zugemischt wird, die 1000 bis 1500 ppm Selen, auf die Trockensubstanz in der Hefe bezogen, entspricht;

b) Isolierens der in Schritt (a) erhaltenen Hefe.

**2.** Verfahren nach Anspruch 1, **gekennzeichnet durch** das Isolieren, einschließlich Ernten, **durch** Zentrifugieren oder Filtrieren.

**3.** Verfahren nach Anspruch 1, **gekennzeichnet** des Weiteren durch das Einschließen der Schritte des:

   c) Waschens der Hefezellen aus Schritt (b),
   d) Hitzebehandelns der Hefezellen aus Schritt (c) und
   e) wahlweisen Trocknens des Produkts aus Schritt (d).

**4.** Verfahren nach einem der Ansprüche 1 bis 3, **dadurch gekennzeichnet, dass** das Minimalmedium aus Rohmaterialien einer pharmazeutischen Qualität besteht.

**5.** Verfahren nach einem der Ansprüche 1 bis 4, **dadurch gekennzeichnet, dass** die Hefe eine Spezies der Gattung *Saccaromycetazeen,* bevorzugt *Saccharomyces cerevisiae, Saccharomyces boulardii sequela* und/oder *Saccharomyces torula* umfasst.

**6.** Verfahren nach Anspruch 5, **dadurch gekennzeichnet, dass** die Hefe *Saccharomyces cerevisiae* ist.

**7.** Selenhefeprodukt, **dadurch gekennzeichnet, dass** es durch das Verfahren nach einem der Ansprüche 1 bis 6 erhältlich ist.

**8.** Verwendung des Selenhefeprodukts nach Anspruch 7 für die Herstellung von Lebensmitteln.

**9.** Verwendung des Selenhefeprodukts nach Anspruch 7 für die Herstellung einer Nahrungsmittelergänzung.

**10.** Verwendung des Selenhefeprodukts nach Anspruch 7 für die Herstellung eines Arzneimittels.

**Revendications**

**1.** Procédé de préparation d'un produit de type levure au sélénium destiné à être utilisé dans un aliment, des compléments alimentaires ou des médicaments, où ladite levure est cultivée sur un milieu minimal dans des conditions aérobies, **caractérisé par** les étapes de :

   a) cultiver la levure, ce qui inclut

   i) des nutriments et du sélénium étant fournis à la levure pendant la culture dans une mesure correspondant à la consommation desdits nutriments et dudit sélénium dans la levure ;
   ii) le glucose et/ou le maltose étant les seules sources de carbone dans le milieu d'alimentation ;
   iii) la concentration d'éthanol pendant la culture ne dépassant pas 1 %, de préférence 0,5 % et de manière particulièrement préférable 0,2 % ;
   iv) le pH pendant la culture étant maintenu entre 4,0 et 6,0, de préférence entre 4,4 et 5,7, de manière particulièrement préférable entre 4,7 et 5,4, par exemple à 5,0 ; et
   v) un sel aqueux de sélénium étant mélangé au milieu d'alimentation en une quantité correspondant à entre 1 000 et 1 500 ppm de sélénium, calculé par rapport à la matière sèche dans la levure ;

   b) isoler la levure obtenue dans l'étape (a).

**2.** Procédé selon la revendication 1, **caractérisé en ce que** l'isolement inclut la récolte par centrifugation ou filtration.

**3.** Procédé selon la revendication 1, **caractérisé en ce qu'**il inclut en outre les étapes de :

   c) laver les cellules de levure provenant de l'étape (b),
   d) traiter thermiquement les cellules de levure provenant de l'étape (c), et
   e) éventuellement sécher le produit provenant de l'étape (d).

**4.** Procédé selon l'une quelconque des revendications 1 à 3, **caractérisé en ce que** le milieu minimal est composé de matières premières d'une qualité pharmaceutique.

5. Procédé selon l'une quelconque des revendications 1 à 4, **caractérisé en ce que** la levure inclut une espèce du genre *Saccharomycetaceae,* de préférence *Saccharomyces cerevisiae, Saccharomyces boulardii sequela* et/ou *Saccharomyces torula.*

6. Procédé selon la revendication 5, **caractérisé en ce que** la levure est *Saccharomyces cerevisiae.*

7. Produit de type levure au sélénium, **caractérisé en ce qu'**il peut être obtenu par le procédé selon l'une quelconque des revendications 1 à 6.

8. Utilisation du produit de type levure au sélénium selon la revendication 7 pour préparer un produit alimentaire.

9. Utilisation du produit de type levure au sélénium selon la revendication 7 pour préparer un complément alimentaire.

10. Utilisation du produit de type levure au sélénium selon la revendication 7 pour préparer un médicament.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4530849 A
- WO 9837172 A
- DK 28507

**Non-patent literature cited in the description**

- **RAYMAN, M.** The importance of selenium to human health. *Lancet,* 2000, vol. 356, 233-241
- *PARNUTS,* 12 May 1999, 5
- *Scientific Committee on Food on the Tolerable Upper Intake Level of Selenium,* 11 October 2000, 2
- A comparison of ten selenium supplementation products. **CLAUSEN, J. et al.** Selenium in Medicine and Biology. Walter de Gruyter & Co, 1988, 305-14
- **SCHRAUZER, G. N.** Selenium in human nutrition. *Bioinorganic Chemistry,* 1978, vol. 8, 303-318
- **LARSEN, E. H. et al.** *J. Anal. At. Spectrom.,* 2000, vol. 16, 1403-1408